# EUROPEAN PATENT SPECIFICATION

(11) **EP 3 666 196 B1**
(45) Date of publication and mention of the grant of the patent: **07.06.2023**
(21) Application number: 19196468.3
(22) Date of filing: 10.09.2019
(51) Int. Cl.: A61B 8/08, A61B 8/00

(54) **ULTRASOUND IMAGING APPARATUS AND COMPUTER PROGRAM PRODUCT**
ULTRASCHALLBILDGEBUNGSVORRICHTUNG UND COMPUTERPROGRAMMPRODUKT
APPAREIL D'IMAGERIE À ULTRASONS ET PRODUIT PROGRAMME INFORMATIQUE

(30) Priority: 12.12.2018 KR 20180160349
(43) Date of publication of application: 17.06.2020
(73) Proprietor: Samsung Medison Co., Ltd., Hongcheon-gun, Gangwon-do, 25108 (KR)
(72) Inventor: GONG, Soyeon, 13530 Gyeonggi-do (KR); PARK, Sungah, 13530 Gyeonggi-do (KR); YANG, Eunho, 13530 Gyeonggi-do (KR); YIM, Yuri, 13530 Gyeonggi-do (KR)
(74) Representative: D'Halleweyn, Nele Veerle Trees Gertrudis

(56) References cited:
- US-A1- 2010 298 704
- US-A1- 2013 338 477
- US-A1- 2015 201 906
- US-A1- 2016 210 511
- US-A1- 2016 338 779

## Description

### BACKGROUND

### 1. Field

The disclosure relates to ultrasound imaging apparatuses, methods of controlling the same, and computer program products.

### 2. Description of Related Art

Ultrasound diagnostic apparatuses transmit ultrasound signals generated by transducers of a probe to an object and detect information about signals reflected from the object, thereby obtaining at least one image of an internal part, for example, soft tissue or blood flow, of the object.

US2016338779 discloses a system including an interventional instrument and an imaging apparatus. The interventional instrument includes an interventional instrument event tracking transmitter that transmits an interventional instrument event signal in response to a predetermined interventional instrument event by interventional instrument in connection with an interventional procedure for a region of interest of a subject. The imaging apparatus includes an imager that generates image of the region of interest of the subject. The imaging apparatus further includes a receiver that receives the interventional instrument event signal. The imaging apparatus further includes an instrument event mapper that maps the predetermined interventional instrument event to the image based on the received interventional instrument event signal.

US2015201906 discloses a medical image diagnostic apparatus comprising a position acquisition unit, a data acquisition unit, an alignment unit, a decision unit, and a data generation unit. The position acquisition unit acquires the position of a medical tool in an imaging region with respect to the imaging region at the time of obtaining the medical image. The data acquisition unit acquires volume data corresponding to a three-dimensional region including the imaging region. The alignment unit decides the position of the volume data. The decision unit decides the position of the medical tool with respect to the volume data based on the position of the medical tool and the position of the volume data. The data generation unit generates support volume data by adding support information indicating the position of the medical tool to the volume data.

US2013338477 discloses a needle assembly including a needle guide, a needle device, a position sensor and additional features. The position sensor allows for the tracking of the needle assembly.

US2016210511 discloses a method including obtaining real-time imaging data of a least a sub-portion of an object and a region of interest therein. The method further includes displaying the real-time imaging data as the real-time imaging is obtained. The method further includes tracking extraction of a sample from the region of interest by an extraction device based on the real-time imaging data. The method further includes identifying an extraction location for the extracted sample based on the tracking and the real-time imaging data and generating a signal indicative thereof.

### SUMMARY

The invention is defined in the independent claim 1 and 5. Preferred embodiments are described in the dependent claims. Provided are methods and apparatuses for automatically storing an ultrasound image when a needle is inserted into an object according to ultrasound image guidance.

Additional aspects will be set forth in part in the description which follows and, in part, will be apparent from the description, or may be learned by practice of the presented embodiments.

The at least one processor may be further configured to detect the first movement of the needle based on position change information of the needle, and the position change information of the needle may be generated based on at least one or a combination of information about a region corresponding to the needle in the real-time ultrasound image, information about a position of the needle, which is received from an external device including the needle, and a signal detected from the needle.

The needle is a needle inserted into the object for FNA.

The at least one processor may be further configured to store in the memory a cine image captured over a predetermined time interval, the cine image including an ultrasound image captured at a time point when the first movement of the needle is detected.

The at least one processor may be further configured to automatically store in the memory the first ultrasound image together with metadata related to the first ultrasound image, and the metadata may include at least one or a combination of information about usage of the needle, information about a particular tissue of the object into which the needle is inserted, and information about a time point when the first movement of the object is detected.

The at least one processor may generate an image file including the first ultrasound image and store the image file in the memory.

In accordance with another aspect of the disclosure not falling within the scope of the invention, a method of controlling an ultrasound imaging apparatus includes: transmitting ultrasound signals to an object and receiving echo signals corresponding to the ultrasound signals; obtaining a real-time ultrasound image of the object from the echo signals; detecting a predefined first movement of a needle inserted into the object; and automatically storing in a memory a first ultrasound image of the object including an ultrasound image captured at a time point when the first movement of the needle is detected.

In accordance with another aspect of the disclosure, a computer program product includes a recording medium having stored therein program instructions for performing a method of controlling an ultrasound imaging apparatus.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a block diagram illustrating an ultrasound diagnosis apparatus according to an exemplary embodiment;
FIGS. 2A, 2B, and 2C are diagrams respectively illustrating an ultrasound diagnosis apparatus according to an embodiment;
FIG. 3 illustrates a method of storing an ultrasound image when a needle is inserted into an object according to ultrasound image guidance, according to an embodiment;
FIG. 4 illustrates a structure of an ultrasound imaging apparatus according to an embodiment;
FIG. 5 is a flowchart of a method of controlling an ultrasound imaging apparatus;
FIG. 6 illustrates an example in which a needle is inserted according to ultrasound image guidance;
FIG. 7 illustrates an example in which an ultrasound imaging apparatus automatically stores an ultrasound image when a needle is advanced into a specific tissue of an object;
FIG. 8 illustrates an example in which an ultrasound imaging apparatus automatically stores an ultrasound image when a needle is repeatedly moved within an object, according to an embodiment;
FIG. 9 illustrates an example in which an ultrasound imaging apparatus automatically stores an ultrasound image when a biopsy needle is shot out of a biopsy gun;
FIG. 10 is a flowchart of a method of controlling an ultrasound imaging apparatus, according to an embodiment;
FIG. 11 illustrates an example in which an ultrasound imaging apparatus sets an ultrasound image automatic storage mode for automatically storing an ultrasound image according to movement of a needle, according to an embodiment; and
FIG. 12 illustrates an example in which an ultrasound imaging apparatus sets a form of movement of a needle as an ultrasound image automatic storage condition in a biopsy mode.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Terms such as "part" and "portion" used herein denote those that may be embodied by software or hardware. According to exemplary embodiments, a plurality of parts or portions may be embodied by a single unit or element, or a single part or portion may include a plurality of elements. Expressions such as "at least one of," when preceding a list of elements, modify the entire list of elements and do not modify the individual elements of the list.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

Hereinafter, embodiments of the disclosure will be described in detail with reference to the accompanying drawings.

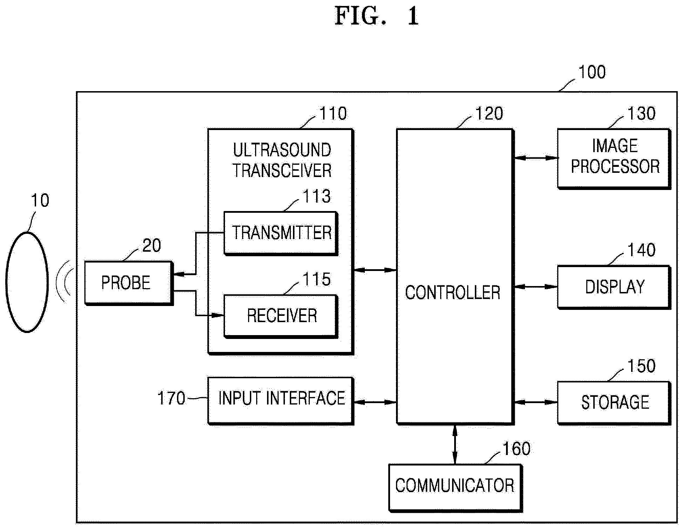

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be of a cart-type or a portable-type ultrasound diagnosis apparatus that is portable, moveable, mobile, or hand-held. Examples of the portable-type ultrasound diagnosis apparatus may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

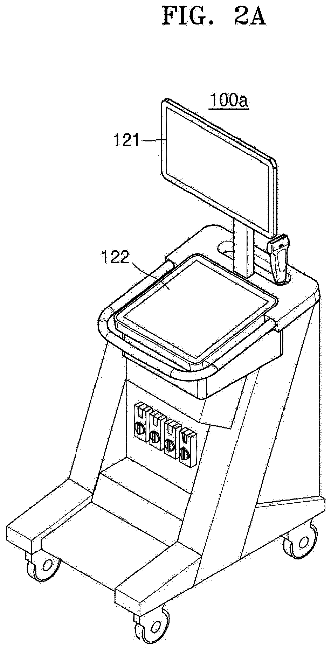

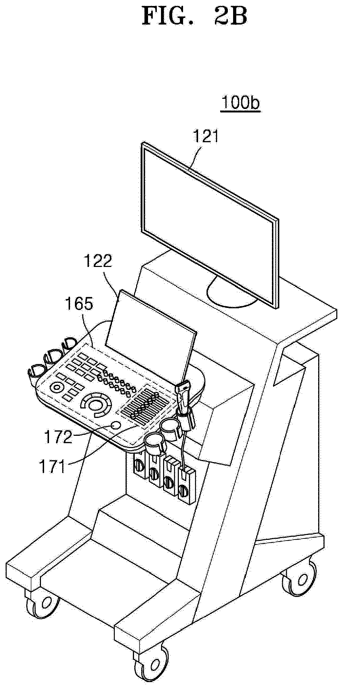

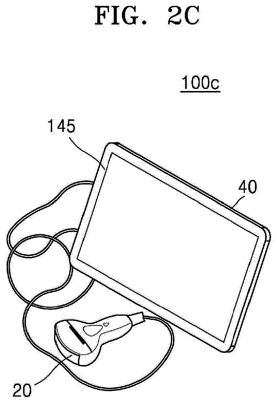

An example of the ultrasound diagnosis apparatus 100 according to the present exemplary embodiment is described below with reference to FIGS. 2A, 2B, and 2C.

FIGS. 2A, 2B, and 2C are diagrams illustrating ultrasound diagnosis apparatus according to an exemplary embodiment.

Referring to FIGS. 2A and 2B, the ultrasound diagnosis apparatus 100 may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100. The main display 121 and the sub-display 122 may provide graphical user interfaces (GUI), thereby receiving user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100 may control the display of the ultrasound image on the main display 121 by using the input control data.

Referring to FIG. 2B, the ultrasound diagnosis apparatus 100 may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100 may include a portable device. Examples of the portable ultrasound diagnosis apparatus may include smart phones including probes and applications, laptop computers, personal digital assistants (PDAs), or tablet PCs, but an exemplary embodiment is not limited thereto.

The ultrasound diagnosis apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100, and a GUI.

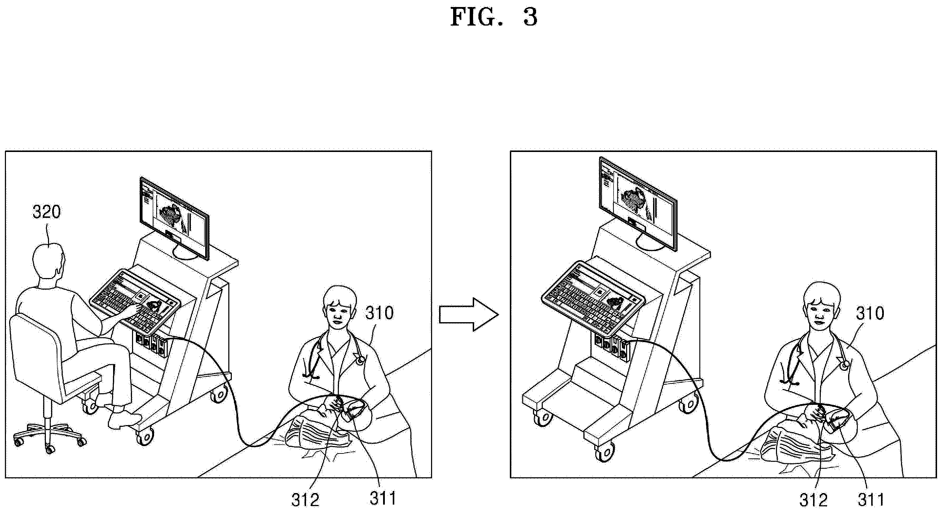

FIG. 3 illustrates a method of storing an ultrasound image when a needle is inserted into an object according to ultrasound image guidance, not falling within the scope of the invention.

A user 310 may insert a needle into an object according to ultrasound image guidance by using an ultrasound imaging apparatus. In this case, the user 310 generally inserts the needle into the object with one hand holding a needle insertion device 312 while acquiring an ultrasound image in real-time with the other hand holding a probe 311. Thus, because the user 310 has to use both hands to inset the needle into the object according to ultrasound image guidance, it is difficult for the user 310 to perform an operation of inserting the needle simultaneously with an operation of storing an ultrasound image. Thus, when the needle is inserted into the object according to ultrasound image guidance, an assistant 320 may be needed to store an ultrasound image related to insertion of the needle.

Accordingly, when a needle is inserted into the object according to ultrasound image guidance, there is a need for methods of storing an ultrasound image related to needle insertion without a help from the assistant 320. The disclosure proposes a method of automatically storing an ultrasound image under a preset condition during insertion of a needle according to ultrasound image guidance. The preset condition may involve detection of a predefined movement of the needle in a real-time ultrasound image of the object. Use of a method of automatically storing a pertinent ultrasound image under a preset condition allows the user 310 to automatically store a desired ultrasound image without a help from the assistant 320.

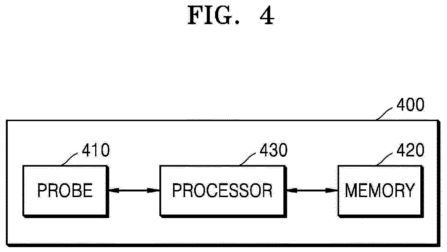

FIG. 4 illustrates a structure of an ultrasound imaging apparatus 400 according to an embodiment.

The ultrasound imaging apparatus 400 according to the embodiment may include a probe 410, a memory 420, and a processor 430.

The probe 410 includes a transducer and transmits ultrasound signals to an object and detects echo signals reflected from the object. The probe 410 may correspond to the probe 20 of FIG. 1.

The memory 420 may store an ultrasound image. The memory 420 may store a file including an ultrasound image. For example, the memory 420 may store a file including an ultrasound image and its associated metadata. According to an embodiment, the memory 420 may be implemented in the form of a main memory, a buffer, a non-volatile memory, an external memory, etc. The memory 420 may include a non-volatile memory for storing an ultrasound image file. For example, a real-time ultrasound image generated based on echo signals may be stored in a main memory, and an ultrasound image automatically stored according to embodiments of the disclosure may be stored in a non-volatile memory via the main memory.

The processor 430 controls all operations of the ultrasound imaging apparatus 400. The processor 430 may be implemented as one or more processors.

The processor 430 may acquire a real-time ultrasound image of an object based on echo signals detected by the probe 410. In this case, acquisition of the ultrasound image may include generation of the ultrasound image based on the echo signals or generation of an ultrasound image file including the ultrasound image generated based on the echo signals.

The processor 430 may detect in the acquired real-time ultrasound image a predefined first movement of a needle inserted into the object. In this case, the needle may include a medical needle that is inserted into the object. For example, the needle may include a biopsy needle shot out of a biopsy gun for collection of tissue of the object, a needle inserted into the object for fine needle aspiration (FNA), a needle for injecting a drug into the object, a needle inserted into the object for radio frequency ablation (RFA), etc. According to the invention the needle is inserted to the object for fine-needle aspiration (FNA).

The first movement of the needle detected by the processor 430 may be a movement of the needle that is set by the ultrasound imaging apparatus 400 as a condition for automatically storing an ultrasound image of the object. For example, the processor 430 may detect in the acquired real-time ultrasound image an advancement of the needle inserted into the object into a specific tissue of the object, a repeated movement of the needle, a movement of the needle at a speed greater than or equal to a predetermined speed, etc. Furthermore, the processor 430 may detect movement of the needle when shot out of the biopsy gun. According to the invention the processor detects a repeated movement of a needle for FNA.

The processor 430 may detect the predefined first movement of the needle based on information about a change in position of the needle inserted into the object (hereinafter, referred to as 'position change information of the needle'). In this case, the position change information of the needle may include information about a region corresponding to the needle, which is acquired through analysis of the real-time ultrasound image, position change information of a needle received from an external device including the needle, and position change information of the needle generated based on an electromagnetic signal detected from the needle.

The processor 430 may automatically store in the memory 420 a first ultrasound image of the object including an ultrasound image captured at a time point when the first movement of the needle is detected. The first ultrasound image may be an ultrasound image that is automatically stored as the first movement is detected and includes an ultrasound image captured at a time point when the first movement is detected. For example, the processor 430 may automatically store in the memory 420 a first ultrasound image including an ultrasound image captured at a time point when an advancement of the needle inserted into the object into a specific tissue of the object is detected. The first ultrasound image may be a cine image captured over a predetermined time interval, the cine image including an ultrasound image captured at a time point when the first movement of the needle is detected. Furthermore, the first ultrasound image may be an ultrasound still image captured at a time point when the first movement of the needle is detected. In this case, detection of the first movement of the needle may be a condition for the processor 430 to automatically store the first ultrasound image.

The processor 430 may set, based on usage of the needle, a form of the first movement of the needle as a condition for automatically storing the first ultrasound image. Furthermore, the processor 430 may set, based on a predefined diagnostic mode, a user input, etc., a form of the first movement of the needle as a condition for automatically storing the first ultrasound image.

The processor 430 may set as an automatic storage condition a first ultrasound image storage mode in correspondence with setting of a form of the first movement of the needle. For example, the first ultrasound image storage mode may include a time interval for the first ultrasound image, a type of a first ultrasound image (e.g., a still image, a cine image, etc.), setting with respect to whether to store associated metadata together, etc.

The processor 430 may generate metadata related to the first ultrasound image that is automatically stored. Furthermore, the processor 430 may store the generated metadata in the memory 420, together with the first ultrasound image. For example, the processor 430 may generate a file including the generated metadata and the first ultrasound image and store the generated file in the memory 420. The metadata related to the first ultrasound image may include at least one or a combination of information about usage of the needle, information about a specific tissue of the object into which the needle is inserted, and information about a time point when the first movement of the object is detected.

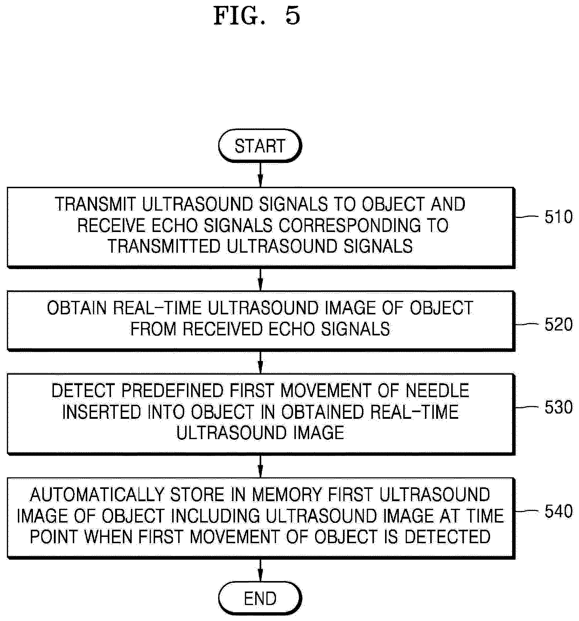

FIG. 5 is a flowchart of a method of controlling an ultrasound imaging apparatus, according to an embodiment.

Operations of the method according to the embodiment may be performed by the ultrasound imaging apparatus 400. The present specification focuses on an embodiment in which the ultrasound imaging apparatus 400 (hereinafter, '400' is a reference numeral used to collectively refer to ultrasound imaging apparatuses described herein) performs a method of controlling an ultrasound imaging apparatus. Thus, embodiments described with respect to the ultrasound imaging apparatus 400 may be applied to a method of controlling an ultrasound imaging apparatus, and embodiments described with respect to the method of controlling an ultrasound imaging apparatus may be applied to the embodiments described with respect to the ultrasound imaging apparatus 400. Methods of controlling an ultrasound imaging apparatus according to embodiments may be performed by the ultrasound imaging apparatus 400, but embodiments are not limited thereto. The methods may be performed by various types of ultrasound imaging apparatuses.

The ultrasound imaging apparatus 400 may transmit ultrasound signals to an object and receive echo signals corresponding to the transmitted ultrasound signals (operation 510).

According to an embodiment, the ultrasound imaging apparatus 400 may use the probe 410 to transmit ultrasound signals to an object and receive echo signals corresponding to the transmitted ultrasound signals. Because transmission of ultrasound signals and reception of echo signals via the probe 410 are already provided above with respect to FIGS. 1, 2, and 4, detailed descriptions thereof will be omitted here.

The ultrasound imaging apparatus 400 may obtain a real-time ultrasound image of the object based on the received echo signals (operation 520).

According to an embodiment, the ultrasound imaging apparatus 400 may generate a real-time ultrasound image of the object based on the received echo signals. Furthermore, the ultrasound imaging apparatus 400 may generate an ultrasound image file including the generated ultrasound image. Hereinafter, acquisition of an ultrasound image may include generation of the ultrasound image based on echo signals or generation of an ultrasound image file including the ultrasound image generated based on the echo signals.

According to an embodiment, the real-time ultrasound image of the object obtained by the ultrasound imaging apparatus 400 may include an image of a specific tissue of the object into which a needle is inserted. For example, the real-time ultrasound image may be an ultrasound image of a specific tissue (e.g., breast, thyroid, etc.) into which a user desires to insert a needle.

According to an embodiment, the real-time ultrasound image of the object obtained by the ultrasound imaging apparatus 400 may be used as a guide image for inserting a needle into the object. The ultrasound imaging apparatus 400 may display the obtained real-time ultrasound image. When a needle is inserted into the object during acquisition of the real-time ultrasound image of the object, the needle inserted into the object may be displayed in the real-time ultrasound image. As the needle appears in the real-time ultrasound image, the user may insert the needle into the object according to a purpose of inserting the needle into the object while viewing the real-time ultrasound image.

According to an embodiment, a needle inserted into the object may be a medical needle for diagnosis, examination, surgical procedure with respect to the object. For example, the needle may include a biopsy needle shot out of a biopsy gun for collection of a living tissue of the object, a needle inserted into the object for FNA, a needle for injecting a drug into the object, a needle inserted into the object for RFA, etc. Hereinafter, the needle may include a medical needle inserted into the object.

The ultrasound imaging apparatus 400 may detect a predefined first movement of a needle inserted into the object in the obtained real-time ultrasound image (operation 530).

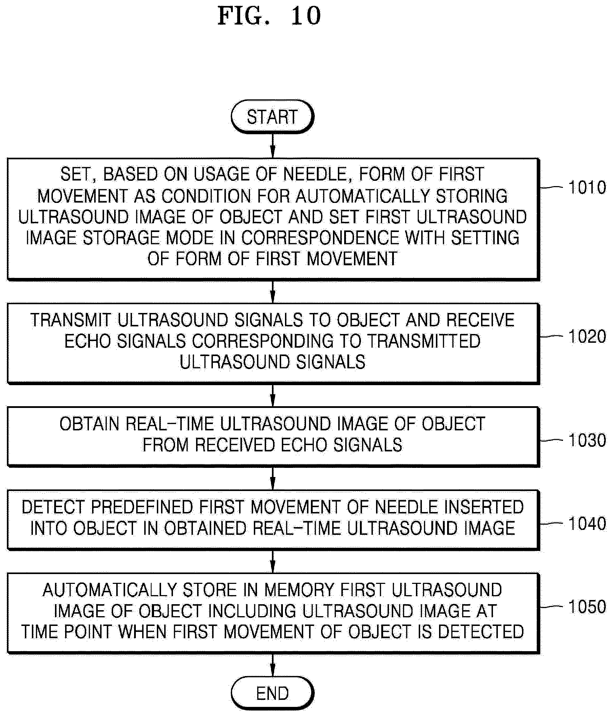

According to an embodiment, the ultrasound imaging apparatus 400 may set a form of a first movement of a needle as a condition for automatically storing an ultrasound image of the object. As the first movement of the needle inserted into the object is detected in the real-time ultrasound image of the object, the ultrasound imaging apparatus 400 may automatically store an ultrasound image of the object. The first movement of the needle hereinafter refers to movement of the needle set by the ultrasound imaging apparatus 400 as a condition for automatically storing an ultrasound image of the object. The first movement may be determined based on at least one or a combination of a form of movement, information about whether to penetrate a surface, a speed of the movement, a magnitude of the movement, a change in a distance from the surface, a type of a moving object, and information about whether to change a direction of the movement. Information about the first movement may be prestored in the ultrasound imaging apparatus 400, or may be generated based on a user input and stored therein. Furthermore, the first movement may be defined differently depending on at least one or a combination of a type of an object, a protocol, a type of biopsy, and a type of a biopsy tool (e.g., a needle). Setting of a form of the first movement of the needle will be described in more detail below in the description of operation 1010 in FIG. 10.

According to an embodiment, the first movement of the needle may include an advancement of the needle into a specific tissue of the object. For example, the first movement of the needle may include a movement of the needle inserted into the object from outside to inside a specific tissue of the object. The movement of the needle from outside to inside the specific tissue of the object may include a movement of the needle in a direction in which a distance between a distal end of the needle and the specific tissue decreases to less than a predetermined value. The ultrasound imaging apparatus 400 may detect as an advancement of the needle into a specific tissue of the object a movement of the needle at a time point when a distance between a distal end of the needle and the specific tissue of the object is less than a predetermined value. In this case, the specific tissue may be a tissue of the object subjected to examination, diagnosis, treatment, etc., using a needle. For example, the specific tissue may be thyroid, breast, or the like.

According to the invention, the first movement of the needle includes a repeated movement of the needle over a predetermined period of time. The repeated movement of the object may include a predetermined movement of the needle that is repeated a predetermined number of times for a specific period of time. For example, the repeated movement of the needle may include a movement that occurs when the needle advancing into a specific tissue of the object repeatedly moves back and forth along an advancement direction over a specific period of time. Furthermore, the repeated movement of the needle may include a repeated movement of the needle for rupturing cells within the object during a specific period of time. In FNA, cells within an object may be ruptured into smaller pieces via a repeated movement of a needle, and the ruptured cells may be aspirated through the needle. The first movement of the needle may include a repeated movement of the needle for rupturing cells within the object during the FNA. However, the repeated movement of the needle is not limited to the repeated movement of the needle for rupturing cells of the object.

According to an embodiment, the first movement of the needle may include a movement of the needle at a speed greater than or equal to a predetermined speed. For example, the first movement of the needle may include a movement of the needle at a speed of 15 mm/sec. A movement of the needle at a speed greater than or equal to a predetermined speed may be set based on a speed of a distal end of the needle. For example, when the distal end of the needle moves at or above the predetermined speed, it may be determined that the needle moves at or above the predetermined speed.

According to an embodiment, when a needle is a biopsy needle shot out of a biopsy gun, a first movement of the needle may include movement of the needle when shot out of a biopsy gun. In this case, the biopsy gun is a medical instrument used to extract a piece of tissue or cells from the object for examination. A biopsy gun may consist of a cannula and a biopsy needle inserted into an object, and the biopsy needle may penetrate through and be combined with the cannula. In other words, the biopsy needle may be an inner needle (or a stylet) combined with the cannula. The biopsy needle may have a notch at its distal end for collecting a piece of tissue of the object. When the cannula is inserted into the object and then the biopsy needle is shot out of the cannula, a piece of tissue of the object may be collected in the notch of the biopsy needle. When the biopsy needle holding the collected piece of tissue is retracted back into the cannula, extraction of the piece of tissue may be completed. Thus, as the biopsy needle is shot out of the biopsy gun, the piece of tissue may be taken from the object. A first movement of the needle may include a movement of a biopsy needle that is fired from a cannula of a biopsy gun, and may be a movement of the needle at a time point when a piece of tissue of the object is extracted.

According to an embodiment, the ultrasound imaging apparatus 400 may generate position change information of the needle. The position change information of the needle may include information about a current position of the needle, information about a direction of movement of the needle, information about a speed of movement of the needle, etc. The position change information of the needle may be generated based on a position of a specific portion of the needle (e.g., a distal end or center of the needle).

According to an embodiment, the position change information of the needle may be generated based on information about a region corresponding to the needle in the real-time ultrasound image, such as brightness information of the region, information about a position of the needle received from an external device, a signal detected from the needle, etc. For example, the ultrasound imaging apparatus 400 may detect a region corresponding to the needle in the real-time ultrasound image and track the detected region to generate position change information of the needle. The region corresponding to the needle may be detected in the real-time ultrasound image by using an ultrasound image analysis technique, and a detailed description of the ultrasound image analysis technique will be omitted here.

According to another embodiment, the ultrasound imaging apparatus may generate position change information of the needle based on information about a position of the needle, which is received from a device including the needle. For example, when the needle is a biopsy needle included in a biopsy gun, the ultrasound imaging apparatus 400 may generate position change information of the needle based on information about a position of the biopsy needle received from the biopsy gun (e.g., information about shooting of the biopsy needle).

According to another embodiment, the ultrasound imaging apparatus 400 may generate positon change information of the needle based on an electromagnetic signal detected from the needle. For example, when the needle inserted into the object is a magnetized needle, the ultrasound imaging apparatus 400 may detect an electromagnetic signal from the magnetized needle. The ultrasound imaging apparatus 400 may then generate position change information of the needle based on a change in the detected electromagnetic signal.

According to an embodiment, the ultrasound imaging apparatus 400 may detect movement of the needle based on the generated position change information of the needle. For example, when the needle moves, the ultrasound imaging apparatus 400 may detect the movement of the needle based on information about a current position of the needle, information about a direction of the movement of the needle, information about a speed of the movement of the needle, etc. Furthermore, the ultrasound imaging apparatus 400 may detect a first movement of the needle in real-time by comparing position change information of the needle with information about a direction of movement of the needle and information about a speed of movement of the needle, which are predefined with respect to the first movement of the needle. In addition, when the needle is a biopsy needle, the ultrasound imaging apparatus400 may detect movement of the needle when shot out of a biopsy gun based on information about shooting of the biopsy needle, which is received from the biopsy gun.

The ultrasound imaging apparatus 400 may automatically store in the memory 420 a first ultrasound image of the object including an ultrasound image captured at a time point when the first movement of the needle is detected (operation 540).

According to an embodiment, an ultrasound image captured at a time point when the first movement of the object is detected may be an ultrasound still image at a time point when the first movement of the needle is detected. For example, when the first movement of the needle is set to an advancement of the needle into a specific tissue of the object, an ultrasound image captured at a time point when the first movement is detected may be an ultrasound still image at a time point when the needle advances into the specific tissue of the object from outside of the specific tissue.

According to an embodiment, when the first movement of the needle is detected in the real-time ultrasound image of the object, the ultrasound imaging apparatus 400 may automatically store in the memory 420 a first ultrasound image including an ultrasound image captured at a time point when the first movement is detected. For example, as an advancement of the needle into a specific tissue of the object is detected in the real-time ultrasound image, the ultrasound imaging apparatus 400 may automatically store in the memory 420 a first ultrasound image including an ultrasound image captured at a time point when the advancement of the needle into the specific tissue of the object is detected. A first ultrasound image hereinafter refers to an ultrasound image that is automatically stored as the first movement is detected and includes an ultrasound still image or ultrasound moving image captured at a time point when the first movement is detected.

According to an embodiment, a first ultrasound image may be a cine image that is captured during a predetermined time interval and includes an ultrasound image captured at a time point when the first movement of the needle is detected. A cine image may be a series of crosssectional images of an internal part of the object, which are arranged sequentially over time, and may be used to examine movement of the internal part of the object. A time interval for a cine image that is automatically stored may be set based on a time point when the first movement of the needle is detected. For example, a time interval for an automatically stored cine image may be set to be a time interval around a time point when the first movement is detected (e.g., a time interval between the time point when the first movement is detected and a time point occurring 5 seconds after the detection time point, or a time interval between time points respectively occurring 5 seconds before and after the detection time point.

According to another embodiment, a first ultrasound image may be an ultrasound still image at a time point when the first movement of the needle is detected. For example, when the first movement of the needle is set to an advancement of the needle into a specific tissue of the object, the ultrasound imaging apparatus 400 may automatically store, as the first movement is detected, an ultrasound still image at a time point when the needle advances into the specific tissue of the object from outside of the specific tissue.

According to an embodiment, the ultrasound imaging apparatus 400 may store a first ultrasound image in a non-volatile memory. For example, the ultrasound imaging apparatus 400 may store the first ultrasound image in a non-volatile memory such as ROM, a flash memory, a magnetic computer memory, an optical disc drive, etc.

According to an embodiment, the ultrasound imaging apparatus 400 may automatically store a first ultrasound image together with its associated metadata. Metadata is data that describes other data, and the metadata stored together with the first ultrasound image may include data related to the first ultrasound image. For example, the metadata stored together with the first ultrasound image may include information about usage of the needle inserted into the object, information about a specific tissue of the object into which the needle is inserted, information about a time point when the first movement of the object is detected, etc. The ultrasound imaging apparatus 400 may generate a single file including the first ultrasound image and its associated metadata and store the generated file in the memory 420.

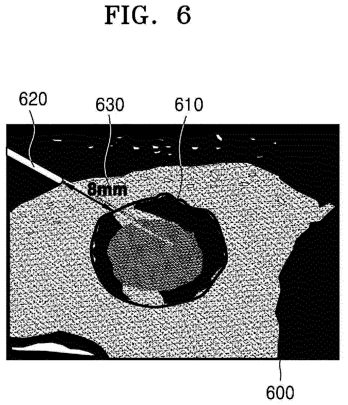

FIG. 6 illustrates an example in which a needle is inserted according to ultrasound image guidance, according to an embodiment.

Referring to FIG. 6, the ultrasound imaging apparatus 400, not falling within the scope of the invention, may obtain and display a real-time ultrasound image 600 of an object. The real-time ultrasound image 600 may be used as a guide image for inserting a needle 620 into the object.

The real-time ultrasound image 600 may show a specific tissue 610 of the object into which the needle 620 is to be inserted. The ultrasound imaging apparatus 400 may detect a region corresponding to the needle 620 inserted into the object in the real-time ultrasound image 600. The ultrasound imaging apparatus 400 may highlight and display the detected region corresponding to the needle 620 in the real-time ultrasound image 600.

The ultrasound imaging apparatus 400 may display information for guiding needle insertion on the real-time ultrasound image 600 in order to guide the needle insertion into the object. For example, the ultrasound imaging apparatus 400 may measure a distance between the needle 620 inserted into the object and a specific tissue 610 of the object and display the measured distance 630 in the real-time ultrasound image 600. Furthermore, the ultrasound imaging apparatus 400 may display on the real-time ultrasound image 600 a guide grid, a guide line indicating a direction of movement of the needle 620, an indicator indicating a distal end of the needle 620, etc., as information for guiding needle insertion.

The ultrasound imaging apparatus 400 may display the real-time ultrasound image 600 and information for guiding needle insertion to allow a user to precisely insert the needle 620 under ultrasound image guidance.

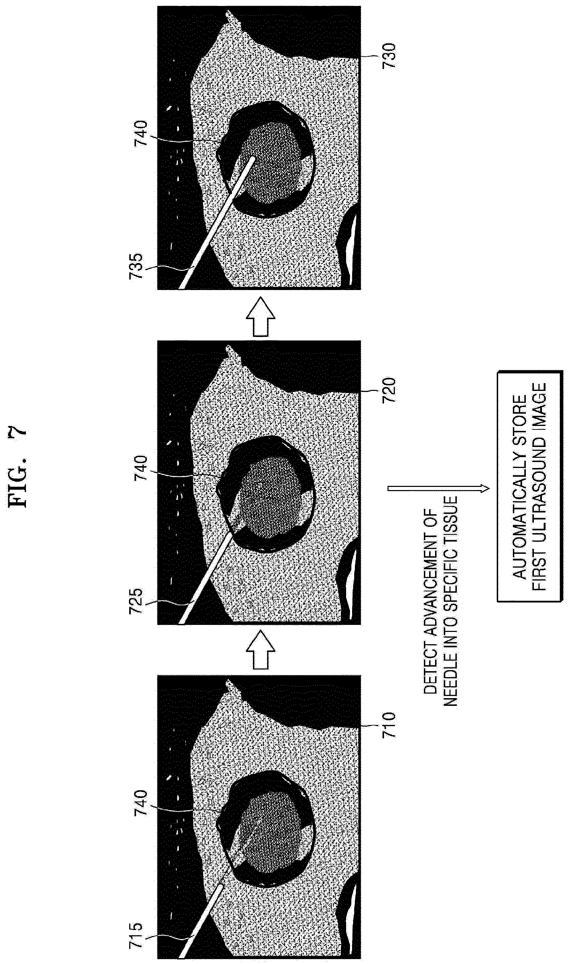

FIG. 7 illustrates an example in the ultrasound imaging apparatus 400, not falling within the scope of the invention, automatically stores an ultrasound image when a needle is advanced into a specific tissue of an object, according to an embodiment.

Referring to FIG. 7, the ultrasound imaging apparatus 400 may obtain and display real-time ultrasound images, i.e., first through third real-time ultrasound images 710, 720, and 730 of the object. The first through third real-time ultrasound images 710, 720, and 730 may each be used as a guide image for inserting a needle into the object and show a specific tissue 740 of the object into which a needle is to be inserted. In this case, the specific tissue 740 may include thyroid, breast, etc., but is not limited thereto.

The ultrasound imaging apparatus 400 may set, as a condition for automatically storing an ultrasound image of an object, a first movement of a needle to an advancement of the needle into the specific tissue 740 of the object. When an advancement of the needle inserted into the object into the specific tissue 740 is detected in the first through third real-time ultrasound images 710, 720, and 730, the ultrasound imaging apparatus 400 may automatically store a first ultrasound image of the object.

Referring to FIG. 7, the first through third real-time ultrasound images 710, 720, and 730 are shown in a chronological order according to the degree of an advancement of needles 715, 725, and 735 into the specific tissue 740. The first real-time ultrasound image 710 shows the needle 715 before being advanced into the specific tissue 740 of the object. The second real-time ultrasound image 720 shows the needle 725 at a time point when the needle 725 is advanced into the specific tissue 740 of the object. The third real-time ultrasound image 730 shows the needle 735 after being advanced into the specific tissue 740 of the object.

The ultrasound imaging apparatus 400 may respectively detect, based on position change information of the needles 715, 725, and 735, movements of the needles 715, 725, and 735 from outside to inside the specific tissue 740 of the object. For example, the ultrasound imaging apparatus 400 may detect in the second real-time ultrasound image 720 a movement of the needle 725 from outside to inside the specific tissue 740 based on information about a current position of the needle 725, information about a direction of movement of the needle 725, etc. In this case, the movement of the needle 725 detected by the ultrasound imaging apparatus 400 may include a movement of the needle 725 in a direction in which a distance between a distal end of the needle 725 and the specific tissue 740 decreases to less than a predetermined value.

As an advancement of a needle into the specific tissue 740 of the object is detected, the ultrasound imaging apparatus 400 may automatically store in the memory 420 a first ultrasound image including the first real-time ultrasound image 720 that is an ultrasound image captured at a time point when the advancement of the needle into the specific tissue 740 is detected. For example, the ultrasound imaging apparatus 400 may store the first real-time ultrasound image 720 as an ultrasound still image, or may store a cine image including the first real-time ultrasound image 720.

The ultrasound imaging apparatus 400 may detect an advancement of a needle inserted into an object into a specific tissue and automatically store an ultrasound image showing the advancement of the needle into the specific tissue as a first ultrasound image. Thus, when the user inserts a needle into a specific tissue of an object under ultrasound image guidance, the ultrasound imaging apparatus 400 may automatically store an ultrasound image showing an advancement of the needle into the specific tissue to allow the user to obtain an ultrasound image related to needle insertion without a help from an assistant.

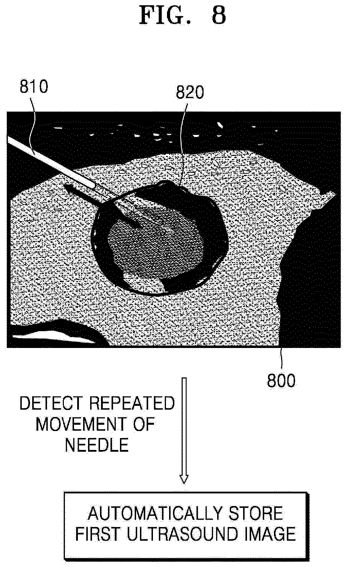

FIG. 8 illustrates an example in which the ultrasound imaging apparatus 400, according to the invention, automatically stores an ultrasound image when a needle repeatedly moves within an object, according to an embodiment.

Referring to FIG. 8, the ultrasound imaging apparatus 400 obtains and displays a real-time ultrasound image 800 of an object. The real-time ultrasound image 800 may be used as a guide image for inserting a needle into the object. The real-time ultrasound image 800 may show a specific tissue 820 of the object that is subjected to examination using needle. In this case, the specific tissue 820 may include thyroid, breast, etc., but is not limited thereto.

The ultrasound imaging apparatus 400 sets, as a condition for automatically storing an ultrasound image of an object, the first movement of a needle 810 to a repeated movement of the needle 810 over a predetermined period of time. When a repeated movement of the needle 810 inserted into the object is detected in the real-time ultrasound image 800, the ultrasound imaging apparatus 400 automatically stores a first ultrasound image of the object.

Referring to FIG. 8, the real-time ultrasound image 800 shows a movement that occurs when the needle 810 advancing into the specific tissue 820 of the object repeatedly moves back and forth along an advancement direction.

The ultrasound imaging apparatus 400 detects, based on position change information of the needle 810 inserted into the object, a movement that occurs when the needle 810 advancing into the specific tissue 820 of the object repeatedly moves back and forth along an advancement direction. For example, the ultrasound imaging apparatus 400 may detect a region corresponding to the needle 810 in the real-time ultrasound image 800 and track the detected region to generate position change information of the needle 810. The ultrasound imaging apparatus 400 may detect a repeated movement of the needle 810 based on the generated position change information of the needle 810.

When the repeated movement of the needle 810 inserted into the object is detected in the real-time ultrasound image 800, the ultrasound imaging apparatus 400 automatically stores a first ultrasound image of the object. For example, the ultrasound imaging apparatus 400 may store the real-time ultrasound image 800 at a time point when the repeated movement of the needle 810 is detected as an ultrasound still image, or may store a cine image including the real-time ultrasound image 800.

The ultrasound imaging apparatus 400 detects a repeated movement of a needle inserted into an object and automatically stores an ultrasound image showing the repeated movement of the needle as a first ultrasound image. Thus, when the user performs FNA using a needle under ultrasound image guidance, the ultrasound imaging apparatus 400 may automatically store an ultrasound image showing a movement of the needle that ruptures cells into small pieces and aspirates the ruptured cells via a repeated movement, thereby allowing the user to obtain an ultrasound image related to needle insertion without a help from an assistant.

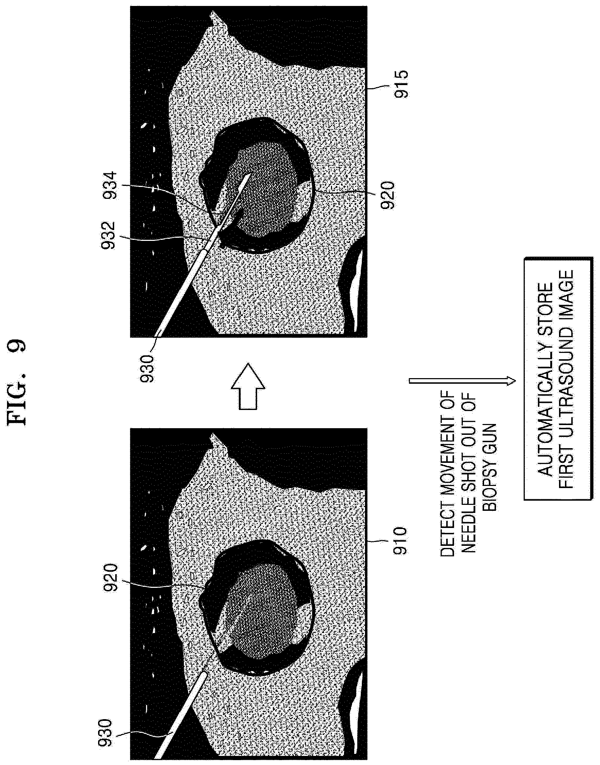

FIG. 9 illustrates an example in which the ultrasound imaging apparatus 400 automatically stores an ultrasound image when a biopsy needle shoots out of a biopsy gun, according to an embodiment.

Referring to FIG. 9, the ultrasound imaging apparatus 400, not falling within the scope of the invention, may obtain and display real-time ultrasound images, i.e., first and second real-time ultrasound images 910 and 915 of an object. The first and second real-time ultrasound images 910 and 915 may be used as a guide image for collecting a piece of tissue of the object by using a biopsy gun. The first and second real-time ultrasound images 910 and 915 may each show a specific tissue 920 of the object that is subjected to examination using a needle. In this case, the specific tissue 920 may include thyroid, breast, etc., but is not limited thereto.

Referring to FIG. 9, the first and second real-time ultrasound images 910 and 915 may each show a movement that occurs when a biopsy needle 932 is fired from a cannula 930 inserted into the object. The first real-time ultrasound image 910 represents an image captured before the biopsy needle 932 is fired from the cannula 930 inserted into the object toward the specific tissue 920. The second real-time ultrasound image 915 represents an image at a time point when the biopsy needle 932 is fired from the cannula 930 inserted into the object.

The ultrasound imaging apparatus 400 may set, as a condition for automatically storing an ultrasound image of an object, a first movement of a needle to a movement of a biopsy needle when shot out of a biopsy gun. In this case, the biopsy needle is included in the biopsy gun and fits into a cannula inserted into the object. A movement of the biopsy needle when shot out of the biopsy gun may include firing of the biopsy needle from the cannula having the biopsy needle engaged therein.

The ultrasound imaging apparatus 400 may detect a movement of the shot biopsy needle 932 based on information about a region corresponding to the cannula 930 and the biopsy needle 932 in the first and second real-time ultrasound images 910 and 915, position change information of the biopsy needle 932 received from the biopsy gun, etc. For example, the ultrasound imaging apparatus 400 may detect a region corresponding to the cannula 930 in the first real-time ultrasound image 910 and track whether the detected region is extended to detect a movement of the biopsy needle 932 fired from the cannula 930. Furthermore, the ultrasound imaging apparatus 400 may receive a signal indicating that the biopsy needle 932 has been shot out of the biopsy gun and detect a movement of the biopsy needle 932 shot out of the biopsy gun based on the received signal.

When movement of the biopsy needle 932 when shot out of the cannula 930 of the biopsy gun is detected in the first and second ultrasound images 910 and 915, the ultrasound imaging apparatus 400 may automatically store a first ultrasound image of the object. For example, the ultrasound imaging apparatus 400 may store a real-time ultrasound image at a time point when the biopsy needle 932 is shot as an ultrasound still image, or may store a cine image including the real-time ultrasound image.

The ultrasound imaging apparatus 400 may automatically store an ultrasound image showing a movement of a biopsy needle when shot out of a biopsy gun as a first ultrasound image. Thus, when the user performs biopsy using a biopsy gun under ultrasound image guidance, the ultrasound imaging apparatus 400 may automatically store an ultrasound image showing collection of a piece of tissue by using the biopsy gun, thereby allowing the user to obtain an ultrasound image related to the biopsy using a needle without a help from an assistant.

FIG. 10 is a flowchart of a method of controlling the ultrasound imaging apparatus 400.

The ultrasound imaging apparatus 400 may set, based on usage of a needle, a form of a first movement of the needle as a condition for automatically storing an ultrasound image of an object and then a first ultrasound image storage mode corresponding to the setting of the form of the first movement of the needle (operation 1010). In this case, as described above, the first movement of the needle may mean a movement of the needle set by the ultrasound imaging apparatus 400 as a condition for automatically storing an ultrasound image of the object.

According to an embodiment, the ultrasound imaging apparatus 400 may set, according to usage of a needle, a form of a first movement of the needle as a condition for automatically storing an ultrasound image of an object. The usage of a needle may be determined according to the type of a medical device including the needle. The needle may be classified into a gun biopsy needle, a FNA biopsy needle, a drug injection needle, a RFA needle, etc. according to its usage. When the needle is a biopsy needle for gun biopsy, the ultrasound imaging apparatus 400 may set a form of the first movement of the needle to a movement of the needle when shot out of a biopsy gun. Furthermore, when the needle is a FNA biopsy needle, the ultrasound imaging apparatus 400 may set a form of the first movement of the needle to a repeated movement of the needle for rupturing tissue of the object into small pieces.

According to another embodiment, the ultrasound imaging apparatus 400 may set, according to its predefined diagnostic mode, a form of a first movement of a needle as a condition for automatically storing an ultrasound image of an object. For example, the ultrasound imaging apparatus 400 may predefine a biopsy mode, a FNA mode, a drug injection mode, a RFA mode, etc., as a diagnostic mode. The ultrasound imaging apparatus 400 may set a form of the first movement of the needle as a condition for automatically storing an ultrasound image of the object according to a diagnostic mode. Furthermore, the ultrasound imaging apparatus 400 may set, based on a user input, a form of the first movement of the needle as a condition for automatically storing an ultrasound image of the object according to a diagnostic mode.

According to an embodiment, the ultrasound imaging apparatus 400 may set, based on a user input, a form of a first movement of a needle as a condition for automatically storing an ultrasound image of an object. For example, the ultrasound imaging apparatus may receive a user input of setting a first movement of a needle to an advancement of the needle into a specific tissue of an object and set a form of the first movement of the needle based on the received user input.

According to an embodiment, the ultrasound imaging apparatus 400 may set a first ultrasound image storage mode in correspondence with setting of a form of a first movement of a needle. The first ultrasound image storage mode may include a time interval for the first ultrasound image, a type of a first ultrasound image (e.g., a still image, a cine image, etc.), setting with respect to whether to store associated metadata together, etc. For example, when an advancement of a needle into a specific tissue of an object is set as a form of a first movement of the needle, the ultrasound imaging apparatus 400 may set a first ultrasound image storage mode to store a cine image having a predetermined time interval around a time point when the first movement is detected (e.g., a time interval between time points respectively occurring 5 seconds before and after the time point when the first movement is detected). Furthermore, the ultrasound imaging apparatus 400 may set metadata related to a first ultrasound image and which is to be stored together with the first ultrasound image (e.g., information about usage of the needle, information about a time point when the first movement of the object is detected, etc.).

The ultrasound imaging apparatus 400 may transmit ultrasound signals to the object and receive echo signals corresponding to the transmitted ultrasound signals (operation 1020). According to an embodiment, operation 1020 may correspond to operation 510 described with reference to FIG. 5.

The ultrasound imaging apparatus 400 may obtain a real-time ultrasound image of the object based on the received echo signals (operation 1030). According to an embodiment, operation 1030 may correspond to operation 520 described with reference to FIG. 5.

The ultrasound imaging apparatus 400 may detect a predefined first movement of a needle inserted into the object in the obtained real-time ultrasound image (operation 1040). According to an embodiment, operation 1040 may correspond to operation 530 described with reference to FIG. 5.

The ultrasound imaging apparatus 400 may automatically store in the memory 420 a first ultrasound image of the object, which includes an ultrasound image captured at a time point when the first movement of the needle is detected (operation 1050). According to an embodiment, operation 1050 may correspond to operation 540 described with reference to FIG. 5.

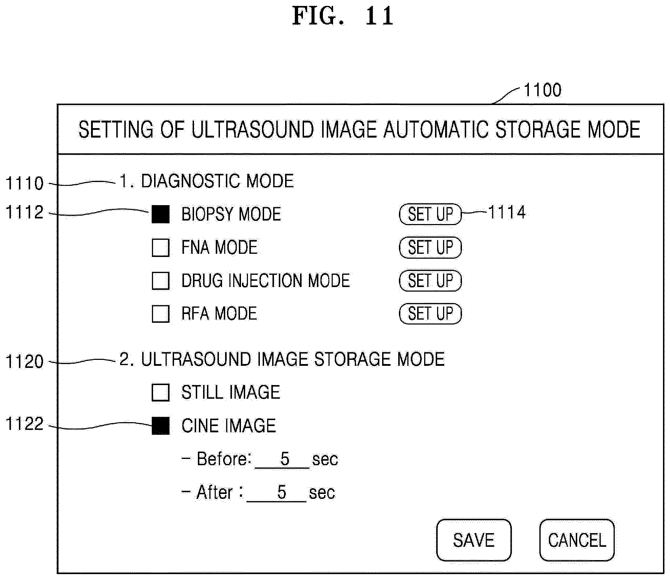

FIG. 11 illustrates an example in which the ultrasound imaging apparatus 400 sets an ultrasound image automatic storage mode for automatically storing an ultrasound image according to a movement of a needle, according to an embodiment.

Referring to FIG. 11, the ultrasound imaging apparatus 400 may display a UI 1100 configured to set a mode for automatically storing an ultrasound image according to a movement of a needle inserted into an object. Setting of an ultrasound image automatic storage mode may include setting of a form of a first movement of a needle, setting of a first ultrasound image storage mode, etc., as a condition for automatically storing an ultrasound image.

The ultrasound imaging apparatus 400 may receive a user input via the displayed UI 1100. The ultrasound imaging apparatus 400 may also set an ultrasound image automatic storage mode based on the user input received via the UI 1100.

The ultrasound imaging apparatus 400 may set, based on the user input, a first movement of a needle as a condition for automatically storing an ultrasound image according to a diagnostic mode. For example, the ultrasound imaging apparatus 400 may predefine an ultrasound image automatic storage mode for each diagnostic mode (a biopsy mode, a FNA mode, a drug injection mode, a RFA mode, etc.) The ultrasound imaging apparatus 400 may set, based on a user input of selecting a diagnostic mode 1110, an ultrasound image automatic storage mode to the selected diagnostic mode1110.

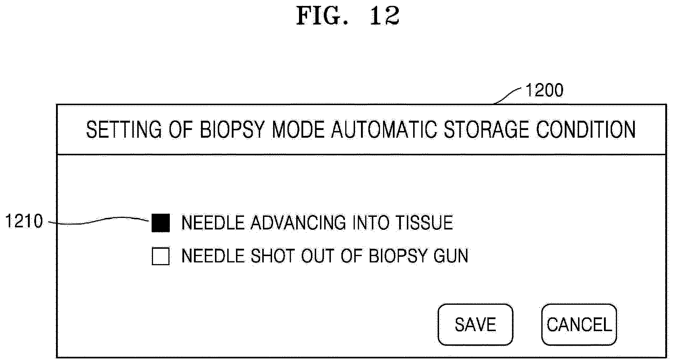

Referring to FIG. 11, the ultrasound imaging apparatus 400 may set, based on a user input of selecting a biopsy mode 1112 as the diagnostic mode 1110, an ultrasound image automatic storage mode so that a movement (i.e., a first movement herein) of a needle is detected in the biopsy mode 1112 under ultrasound image guidance and then an ultrasound image is automatically stored. In this case, the first movement of the needle as an automatic storage condition in the biopsy mode 1112 may be predefined, or may be set based on a user input. For example, the ultrasound imaging apparatus 400 may display, based on a user input of subsequently setting the first movement (1114), a UI for setting the first movement as a condition for automatically storing an ultrasound image in the biopsy mode 1112. Referring to FIG. 12, the ultrasound imaging apparatus 400, not falling within the scope of the invention, may set, based on a user input of setting a first movement via a UI 1200, the first movement as an automatic storage condition in a biopsy mode. The ultrasound imaging apparatus 400 may set an advancement 1210 of a needle into a specific tissue of an object as the first movement.

The ultrasound imaging apparatus 400 may set a first ultrasound image storage mode based on a user input. For example, the ultrasound imaging apparatus 400 may set a type of an ultrasound image to be automatically stored. Referring to FIG. 11, the ultrasound imaging apparatus 400 may set, based on a user input, a type of an ultrasound image to be automatically stored to a cine image 1122. Furthermore, when the cine image 1122 is stored as a first ultrasound image, the ultrasound imaging apparatus 400 may set a time interval for the cine image 1122 based on the user input. For example, the ultrasound imaging apparatus 400 may set the time interval to be between time points respectively occurring 5 seconds before and after the first movement is detected.

According to an embodiment, the ultrasound imaging apparatus 400 may set a time interval for the cine image 1122 to be up to a time point when a defined first movement is detected. For example, when the first movement is a reciprocating movement of the needle, the ultrasound imaging apparatus 400 may store the cine image 1122 while the reciprocating movement of the needle is detected.

Methods of controlling an ultrasound imaging apparatus according to embodiments may be recorded in non-transitory computer-readable recording media having stored therein one or more program including instructions for executing the methods. Examples of non-transitory computer-readable recording media include magnetic media such as hard disks, floppy disks, and magnetic tape, optical media such as CD-ROM and DVDs, magneto-optical media such as floptical discs, and hardware devices that are specially configured to store and perform program instructions, such as ROM, RAM, flash memory, and the like. Examples of program instructions include not only machine code such as that created by a compiler but also high level language code that may be executed by a computer using an interpreter or the like.

While one or more embodiments have been described with reference to the figures, it will be understood by those of ordinary skill in the art that the embodiments are not to be construed as limiting the scope of the disclosure. Various changes and modifications in form and details based on the basic concept of the disclosure may fall within the scope as defined by the following claims.

## Claims

1. An ultrasound imaging apparatus (100,400) comprising:
a probe (20,311,410) configured to transmit ultrasound signals to an object and receive echo signals corresponding to the ultrasound signals;
a memory (420); and
at least one processor (130,430) configured to:
obtain a real-time ultrasound image of the object from the echo signals,
detect a predefined first movement of a needle (810) based on position change information of the needle, and
automatically store in a memory (420) a first ultrasound image (710,910) of the object including an ultrasound image captured at a time point when the first movement of the needle is detected,
wherein the needle comprises a needle inserted into the object for fine needle aspiration (FNA),
wherein the first movement of the needle comprises a repeated movement of the needle for the FNA over a predetermined period of time, and
wherein the at least one processor (130,430) is further configured to automatically store in the memory the first ultrasound image including an ultrasound image captured at a time point when the repeated movement of the needle for the FNA is detected.

2. The ultrasound imaging apparatus (100,400) of claim 1, wherein the at least one processor (130,430) is further configured to set, based on usage of the needle, a form of the first movement of the needle as a condition for automatically storing the first ultrasound image (910) and set a first ultrasound image storage mode in correspondence with the setting.

3. The ultrasound imaging apparatus of claim 1,
wherein the position change information of the needle is generated based on at least one or a combination of information about a region corresponding to the needle in the ultrasound image, information about a position of the needle, which is received from an external device including the needle, and a signal detected from the needle.

4. The ultrasound imaging apparatus (100,400) of claim 1, wherein the at least one processor (130,430) is further configured to store in the memory a cine image captured over a predetermined time interval, the cine image including an ultrasound image captured at a time point when the first movement of the needle is detected.

5. A computer program product comprising a recording medium having stored therein program instructions which, when executed by a processor of an ultrasound imaging apparatus , perform a method of controlling the ultrasound imaging apparatus, the method comprising:
transmitting ultrasound signals to an object and receiving echo signals corresponding to the ultrasound signals;
obtaining a real-time ultrasound image of the object from the echo signals;
detecting a predefined first movement of a needle based on position change information of the needle; and
automatically storing in a memory a first ultrasound image of the object including an ultrasound image captured at a time point when the first movement of the needle is detected,
wherein the needle comprises a needle inserted into the object for fine needle aspiration(FNA),
wherein the first movement of the needle comprises a repeated movement of the needle for the FNA over a predetermined period of time, and
wherein the automatically storing of the first ultrasound image in the memory comprises automatically storing the first ultrasound image including an ultrasound image captured at a time point when the repeated movement of the needle for the FNA is detected.

## Patentansprüche

1. Ultraschallbildgebungsvorrichtung (100, 400), die Folgendes umfasst:
eine Sonde (20, 311, 410), die dazu konfiguriert ist, Ultraschallsignale an ein Objekt zu senden und den Ultraschallsignalen entspreche Echosignale zu empfangen;
einen Speicher (420); und
mindestens einen Prozessor (130, 430), der zu Folgendem konfiguriert ist:
Erhalten eines Echtzeit-Ultraschallbildes des Objekts aus den Echosignalen, Erfassen einer vordefinierten ersten Bewegung einer Nadel (810) basierend auf Positionsänderungsinformationen der Nadel, und
in einem Speicher (420), automatisches Speichern eines ersten Ultraschallbildes (710, 910) des Objekts, einschließlich eines Ultraschallbildes, das zu einem Zeitpunkt aufgenommen wurde, an dem die erste Bewegung der Nadel erfasst wurde,
wobei die Nadel eine in das Objekt eingeführte Nadel zur Feinnadelaspiration (FNA) umfasst,
wobei die erste Bewegung der Nadel eine wiederholte Bewegung der Nadel für die FNA über einen vorbestimmten Zeitraum umfasst, und
wobei der mindestens eine Prozessor (130, 430) ferner dazu konfiguriert ist, das erste Ultraschallbild, einschließlich eines Ultraschallbildes, das zu einem Zeitpunkt aufgenommen wurde, an dem die wiederholte Bewegung der Nadel für die FNA erfasst wurde, automatisch in dem Speicher zu speichern.

2. Ultraschallbildgebungsvorrichtung (100, 400) nach Anspruch 1, wobei der mindestens eine Prozessor (130, 430) ferner dazu konfiguriert ist, basierend auf der Verwendung der Nadel eine Form der ersten Bewegung der Nadel als eine Bedingung für das automatische Speichern des ersten Ultraschallbildes (910) einzustellen und einen ersten Ultraschallbild-Speichermodus entsprechend der Einstellung einzustellen.

3. Ultraschallbildgebungsvorrichtung nach Anspruch 1, wobei die Positionsänderungsinformationen der Nadel basierend auf mindestens einem oder einer Kombination der Folgenden erzeugt werden: Informationen über eine der Nadel entsprechende Region in dem Echtzeit-Ultraschallbild, Informationen über eine Position der Nadel, die von einer die Nadel enthaltenden externen Vorrichtung empfangen werden, und ein aus der Nadel stammendes, erfasstes Signal.

4. Ultraschallbildgebungsvorrichtung (100, 400) nach Anspruch 1, wobei der mindestens eine Prozessor (130, 430) ferner konfiguriert ist, um in dem Speicher ein Cine-Bild zu speichern, das über ein vorbestimmtes Zeitintervall aufgenommen wurde, wobei das Cine-Bild ein Ultraschallbild enthält, das zu einem Zeitpunkt aufgenommen wurde, zu dem die erste Bewegung der Nadel erfasst wurde.

5. Computerprogrammprodukt, umfassend ein Aufzeichnungsmedium mit darin gespeicherten Programmanweisungen, die, wenn sie von dem Prozessor einer Ultraschallbildgebungsvorrichtung ausgeführt werden, ein Verfahren zum Steuern der Ultraschallbildgebungsvorrichtung ausführen, wobei das Verfahren Folgendes umfasst:
Senden von Ultraschallsignalen an ein Objekt und Empfangen von den Ultraschallsignalen entsprechenden Echosignalen;
Erhalten eines Echtzeit-Ultraschallbildes des Objekts aus den Echosignalen;
Erfassen einer vordefinierten ersten Bewegung einer Nadel basierend auf Positionsänderungsinformationen der Nadel; und
in einem Speicher, automatisches Speichern eines ersten Ultraschallbildes des Objekts, einschließlich eines Ultraschallbildes, das zu einem Zeitpunkt aufgenommen wurde, an dem die erste Bewegung der Nadel erfasst wurde,
wobei die Nadel eine in das Objekt eingeführte Nadel zur Feinnadelaspiration (FNA) umfasst,
wobei die erste Bewegung der Nadel eine wiederholte Bewegung der Nadel für die FNA über einen vorbestimmten Zeitraum umfasst, und
wobei das automatische Speichern des ersten Ultraschallbildes in dem Speicher Folgendes umfasst: automatisches Speichern des ersten Ultraschallbildes einschließlich eines Ultraschallbildes, das zu einem Zeitpunkt aufgenommen wurde, an dem die wiederholte Bewegung der Nadel für die FNA erfasst wurde.

## Revendications

1. Appareil d'imagerie échographique (100, 400) comprenant :
une sonde (20, 311, 410) conçue pour émettre des signaux ultrasonores en direction d'un objet et recevoir des signaux d'écho correspondant aux signaux ultrasonores,
une mémoire (420), et
au moins un processeur (130, 430) conçu pour :
obtenir une image échographique en temps réel de l'objet à partir des signaux d'écho,
détecter un premier mouvement prédéfini d'une aiguille (810) compte tenu d'informations de changement de position de l'aiguille, et
stocker automatiquement dans une mémoire (420) une première image échographique (710, 910) de l'objet comprenant une image échographique réalisée à l'instant auquel le premier mouvement de l'aiguille est détecté ;
ladite aiguille comprenant une aiguille introduite dans l'objet pour aspiration à l'aiguille fine (AAF),
ledit premier mouvement de l'aiguille comprenant un mouvement répété de l'aiguille pour AAF sur une période prédéterminée, et
ledit au moins un processeur (130, 430) étant conçu en outre pour stocker automatiquement dans la mémoire la première image échographique comprenant une image échographique réalisée à l'instant auquel le mouvement répété de l'aiguille pour AAF est détecté.

2. Appareil d'imagerie échographique (100, 400) selon la revendication 1, dans lequel l'au moins un processeur (130, 430) est conçu en outre pour régler, compte tenu de l'usage de l'aiguille, la forme du premier mouvement de l'aiguille comme condition de stockage automatique de la première image échographique (910) et pour régler un mode de stockage de la première image échographique en correspondance avec le réglage.

3. Appareil d'imagerie échographique selon la revendication 1,
dans lequel les informations de changement de position de l'aiguille sont générées compte tenu d'au moins un ou d'une combinaison des éléments suivants : des informations concernant une région correspondant à l'aiguille dans l'image échographique en temps réel, des informations concernant la position de l'aiguille, reçues en provenance d'un appareil externe comportant l'aiguille, et un signal détecté en provenance de l'aiguille.

4. Appareil d'imagerie échographique (100, 400) selon la revendication 1, dans lequel l'au moins un processeur (130, 430) est conçu en outre pour stocker, dans la mémoire, une image en mode ciné capturée sur une durée prédéterminée, ladite image en mode ciné comprenant une image échographique réalisée à l'instant auquel le premier mouvement de l'aiguille est détecté.

5. Produit-programme informatique comprenant un support d'enregistrement sur lequel sont enregistrées des instructions de programme qui, lorsqu'elles sont exécutées par le processeur d'un appareil d'imagerie échographique, réalisent un procédé de commande de l'appareil d'imagerie échographique, ledit procédé comprenant :
l'émission de signaux ultrasonores en direction d'un objet et la réception de signaux d'écho correspondant aux signaux ultrasonores,
l'obtention d'une image échographique en temps réel de l'objet à partir des signaux d'écho,
la détection d'un premier mouvement prédéfini d'une aiguille compte tenu d'informations de changement de position de l'aiguille, et
le stockage automatique, dans une mémoire, d'une première image échographique de l'objet comprenant une image échographique réalisée à l'instant auquel le premier mouvement de l'aiguille est détecté ;
ladite aiguille comprenant une aiguille introduite dans l'objet pour aspiration à l'aiguille fine (AAF),
ledit premier mouvement de l'aiguille comprenant un mouvement répété de l'aiguille pour AAF sur une période prédéterminée, et
ledit stockage automatique de la première image échographique dans la mémoire comprenant le stockage automatique de la première image échographique comprenant une image échographique réalisée à l'instant auquel le mouvement répété de l'aiguille pour AAF est détecté.
